# EUROPEAN PATENT APPLICATION

(11) **EP 3 375 420 A1**
(43) Date of publication of application: **19.09.2018**
(21) Application number: 18159805.3
(22) Date of filing: 02.03.2018
(51) Int. Cl.: A61F 13/20, A61L 15/60, A61F 13/36

(54) **DENTAL ABSORBENT PAD**

(30) Priority: 03.03.2017 US 201762466422 P
(71) Applicant: Microcopy Ltd., 18110 Afula (IL)
(72) Inventor: Parke, Perry L., Kennesaw, GA Georgia 30156 (US); Brayman, Emily, Kennesaw, GA Georgia 30156 (US)
(74) Representative: J A Kemp

(57) **Abstract**

A dental absorbent pad (10) has a moisture permeable outer layer (11) which is folded upon itself to form a top side (12), a bottom side (13), and a peripheral margin area (15). The folded outer layer forms a cavity between the top side and the bottom side which is filled with a mass of unsupported, free-flowing granular superabsorbent material (16). The mass of granular superabsorbent material may be grains of sodium salt of cross linked polyacrylic acid with a grain size or diameter of 1 to 850 microns.

## Description

### REFERENCE TO RELATED APPLICATION

Applicant claims the benefit of U.S. Provisional Patent Application Serial No. 62/466,422 filed March 3, 2017 and entitled Dental Absorbent Pad.

### TECHNICAL FIELD

This invention relates generally to absorbent pads, and particularly absorbent pads which are used in the dental industry.

### BACKGROUND OF THE INVENTION

Small absorbent pads are often used in dentistry to absorb saliva from the mouth of a patient during a dental procedure. These pads are typically positioned between the teeth and cheek or between the teeth and tongue and are replaced when they become saturated.

The most common type of dental absorbent pad is simply a mass or roll of cotton. While these cotton rolls may provide absorbency, they typically expand with moisture and may become difficult to properly position.

The most effective dental absorbent pads typically have a moisture permeable outer layer made of a nylon fabric, a super absorbent polymer core, and a moisture impermeable, second outer layer of polyethylene film. These materials are held together with a thick layer of hardened, hot-melt adhesive. This hardened adhesive however causes the peripheral edges of the pad to be stiff and abrasive, which may cause irritation to the tissue of the mouth.

Another type of dental absorbent pad is shown in patent publication WO2014003642 A1. Here the absorbent pad has a liquid transporting wrapping which encloses a superabsorbent material. The superabsorbent material is typically a sheet of cotton material which supports granules of a superabsorbent polymer embedded within the cotton material. A problem with this type of device is that the granules of superabsorbent polymer are released by the cotton material and migrate through the liquid transporting wrapping. Thus, the superabsorbent polymer grains are released into the mouth of the patient. Also, this product is restricted in shape to generally that of the sheet of cotton material which provides the structural integrity or support of the dental absorbent pad and may not conform with an individual's mouth.

Accordingly, it is seen that a need remains for a dental absorbent pad which is more comfortable or conformable for a patient. It is to the provision of such that the present invention is primarily directed.

### SUMMARY OF THE INVENTION

A dental absorbent pad comprises a moisture permeable outer layer defining an interior cavity, and a mass of free-flowing, granular moisture absorbent material positioned within the cavity.

The dental absorbent pad optionally further comprises an adhesive layer coupled to an exterior surface of a portion of said moisture permeable outer layer.

Optionally, said mass of free-flowing, granular absorbent material is made of a granular sodium salt of cross linked polyacrylic acid.

Optionally, said granular sodium salt of cross linked polyacrylic acid has a grain diameter of 1 to 850 microns.

Optionally, said mass of free flowing, granular absorbent material has a grain size of 1 to 850 microns.

Optionally, wherein said outer layer is a layer of polypropylene material with a hydrophilic coating.

A dental absorbent pad comprises a moisture permeable outer layer folded upon itself to form a first layer and a second layer with a space between said first layer and said second layer, and a mass of free-flowing moisture absorbent material positioned within said space.

The dental absorbent pad optionally further comprises an adhesive layer coupled to an exterior surface of one said layer of said moisture permeable outer layer.

Optionally, said mass of free-flowing absorbent material is made of a granular sodium salt of cross linked polyacrylic acid.

Optionally, said granular sodium salt of cross linked polyacrylic acid has a grain diameter of 1 to 850 microns.

Optionally, said mass of free flowing absorbent material is granular and has a grain size of 1 to 850 microns.

Optionally, said outer layer is a layer of polypropylene material with a hydrophilic coating.

A dental absorbent pad comprises an outer envelope having at least a portion being permeable to saliva, said outer envelope surrounding a mass of unsupported, granular moisture absorbent material.

The dental absorbent pad optionally further comprises an adhesive layer coupled to an exterior surface of a portion of said outer envelope.

Optionally, said mass of unsupported, granular absorbent material is made of a granular sodium salt of cross linked polyacrylic acid.

Optionally, said granular sodium salt of cross linked polyacrylic acid has a grain diameter of 1 to 850 microns.

Optionally, said mass of unsupported, granular absorbent material has a grain size of 1 to 850 microns.

Optionally, said outer envelope is a layer of polypropylene material with a hydrophilic coating.

### BRIEF DESCRIPTION OF THE DRAWINGS

Fig. 1 is a perspective view of a dental absorbent pad in a preferred form of the invention.
Fig. 2 is a cross-sectional view of the dental absorbent pad of Fig. 1.
Fig. 3 is a top view of a dental absorbent pad in another embodiment of the invention.
Fig. 4 is a top view of a dental absorbent pad in another embodiment of the invention.
Fig. 5 is a perspective view of a dental absorbent pad in another embodiment of the invention.

### DETAILED DESCRIPTION

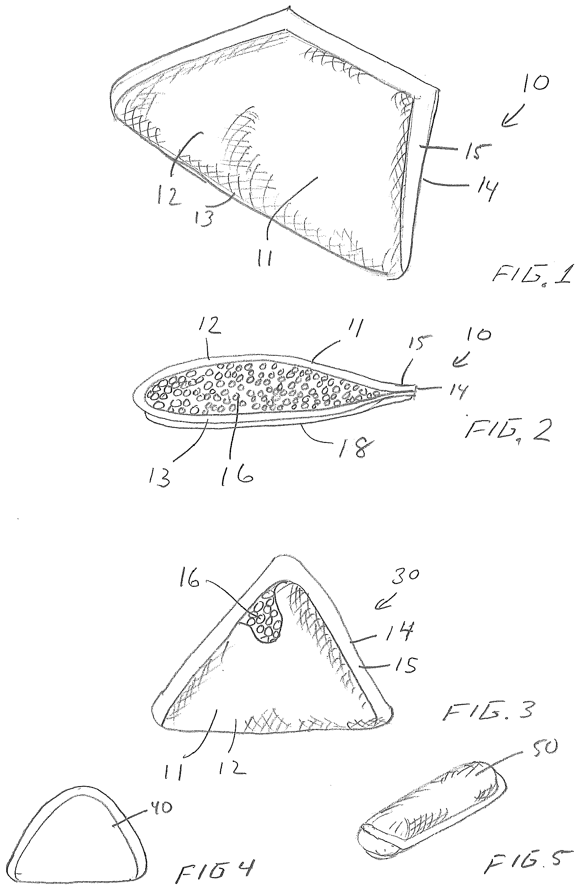

With reference next to the drawings, there is shown in Figs. 1 and 2 a dental absorbent pad 10 embodying principles of the invention in a preferred form. The dental absorbent pad 10 optionally has a moisture permeable cover or outer layer 11 which is optionally folded upon itself to form an envelope with a top side 12, and a bottom side 13. The folded outer layer 11 is optionally bonded along its unfolded peripheral edge 14 to form a margin area 15. The dental absorbent pad 10 optionally has a generally trapezoidal shape, however other shapes may be utilized in addition to the variations described herein which are believed to fit within the mouth under the tongue (sublingual) better and provide more comfort than the crescent shape of the prior art pads. These other shapes may include crescent shapes, hyperbolas, pillow-shapes, cylinders, or the like.

The folded outer layer 11 optionally forms a cavity or space between the top side 12 and the bottom side 13 which is filled with a mass of unsupported, free-flowing granular superabsorbent material 16. The term mass of unsupported or free-flowing granular material is intended to denote a mass which is comprised of granular material which is allowed to move or flow freely, rather than granular material which is restricted or constrained from movement by a supporting or secondary material, i.e., a mass of granular material which is essentially void of a secondary material.

The mass of granular superabsorbent material 16 may be grains of sodium salt of cross linked polyacrylic acid. The granular material may have a grain size or diameter of 1 to 850 microns. The outer layer 11 is preferably made of a material having interstices sized to contain the granular superabsorbent material yet allow the passage of saliva. The outer layer 11 may be made of a micro-thin high density woven or non-woven netting. For example, the outer layer 11 may be a non-woven, spunbond polypropylene material with a hydrophilic coating.

It should be understood that as the margin area 15 of the pad is preferably formed from very thin and flexible layers of material which are heat welded, or otherwise welded or bonded, and is not considered to be stiff, as opposed to prior art pads. Furthermore, the reduction in stiffness and thickness of this peripheral area reduces the abrasiveness of the peripheral edge against the tissues of the mouth, thereby further increasing the comfort of the present invention over prior art dental absorbent pads.

It should be understood that the composition of the material is not intended to be limited by those recited herein and may be different from that specifically used in the preferred embodiment. For example, the outer layer 11 may be made of a woven, non-woven or porous polymer material, such as a woven nylon or non-woven fiber. Similarly, the superabsorbent material 16 may be made of grains of other types of conventionally known superabsorbent material and may be supplemented with other granular filler materials.

As an option, the absorbent pad 10 may also include a layer of dry adhesive 18 upon the exterior surface of the bottom side 13. The adhesive layer 18 helps maintain the position of the absorbent pad 10 within the mouth during use by adhering the absorbent pad 10 to the tissue of the patient's mouth. The adhesive layer 18 is activated through contact with saliva, water or other liquids.

By utilizing a mass of free-flowing granules the dental pad may more easily take on a shape conforming to the interior of a patient's mouth. This conforming ability provides a better and more comfortable fit for the dental pad during use, as opposed to a pad having an interior support structure to hold granules of absorbent material.

In use, the absorbent pad 10 is placed within the patient's mouth. Saliva within this area passes through the outer layer 11 and is absorbed by the granular superabsorbent material 16. The absorption of the saliva does not significantly increase the size or volume of the absorbent pad 10.

With reference next to the embodiment shown in Fig. 3, here the dental absorbent pad 30 is in essence the same as that shown in Figs. 1 and 2 except for the overall shape. The dental absorbent pad 30 has a generally rounded cornered, triangular shape. The dental absorbent pad 30 includes the outer layer 11, margin area 15, and cavity filled with a mass of free-flowing granular superabsorbent material 16.

With reference next to the embodiment shown in Fig. 4, here the dental absorbent pad 40 is in essence the same as that shown in Figs. 1 and 2 except for the overall shape. The dental absorbent pad 40 has a generally crescent or arcuate shape. The dental absorbent pad 40 includes the outer layer 11, margin area 15, and cavity filled with a mass of free-flowing granular superabsorbent material 16.

With reference next to the embodiment shown in Fig. 5, here the dental absorbent pad 50 is in essence the same as that shown in Figs. 1 and 2 except for the overall shape. The dental absorbent pad 50 has a generally pillow or somewhat cylindrical shape. The dental absorbent pad 50 includes the outer layer 11, margin area 15, and cavity filled with a mass of free-flowing granular superabsorbent material 16.

It thus is seen that a dental absorbent pad is now provided which is more comfortable for a patient. While this invention has been described in detail with particular references to the preferred embodiments thereof, it should be understood that many modifications, additions and deletions, in addition to those expressly recited, may be made thereto without departure from the scope of the invention as set forth in the following claims.

## Claims

1. A dental absorbent pad comprising:
a moisture permeable outer layer defining an interior cavity, and
a mass of free-flowing moisture absorbent material positioned within said cavity.

2. The dental absorbent pad of claim 1, wherein said mass of free-flowing moisture absorbent material is granular.

3. The dental absorbent pad of claim 1 or claim 2, further comprising an adhesive layer coupled to an exterior surface of a portion of said moisture permeable outer layer.

4. The dental absorbent pad of any one of the preceding claims, wherein said mass of free-flowing absorbent material is made of a granular sodium salt of cross linked polyacrylic acid.

5. The dental absorbent pad of claim 4 wherein said granular sodium salt of cross linked polyacrylic acid has a grain diameter of 1 to 850 microns.

6. The dental absorbent pad of any one of the preceding claims, wherein said mass of free flowing absorbent material is granular and has a grain size of 1 to 850 microns.

7. The dental absorbent pad of any one of the preceding claims, wherein said outer layer is a layer of polypropylene material with a hydrophilic coating.

8. The dental absorbent pad of any one of the preceding claims, wherein said moisture permeable outer layer is folded upon itself to form a first layer and a second layer with a space between said first layer and said second layer, and wherein said mass of free-flowing moisture absorbent material is positioned within said space.

9. The dental absorbent pad of claim 8, further comprising an adhesive layer coupled to an exterior surface of one said layer of said moisture permeable outer layer.

10. A dental absorbent pad comprising:
an outer envelope having at least a portion being permeable to saliva, said outer envelope surrounding a mass of unsupported, granular moisture absorbent material.

11. The dental absorbent pad of claim 10, further comprising an adhesive layer coupled to an exterior surface of a portion of said outer envelope.

12. The dental absorbent pad of claim 10 or claim 11, wherein said mass of unsupported, granular absorbent material is made of a granular sodium salt of cross linked polyacrylic acid.

13. The dental absorbent pad of claim 12, wherein said granular sodium salt of cross linked polyacrylic acid has a grain diameter of 1 to 850 microns.

14. The dental absorbent pad of any one of claims 10 to 13, wherein said mass of unsupported, granular absorbent material has a grain size of 1 to 850 microns.

15. The dental absorbent pad of any one of claims 10 to 14, wherein said outer envelope is a layer of polypropylene material with a hydrophilic coating.
